# EUROPÄISCHE PATENTANMELDUNG

(11) **EP 0 531 822 A1**
(43) Veröffentlichungstag der Anmeldung: **17.03.1993**
(21) Anmeldenummer: 92114685.8
(22) Anmeldetag: 28.08.1992
(51) Int. Cl.: A61B 17/22

(54) **Vorrichtung zum Entfernen von organischen Materialien aus dem menschlichen Körper**

(30) Priorität: 03.09.1991 DE 9110902 U
(71) Anmelder: ANGIOMED AG, D-76227 Karlsruhe (DE)
(72) Erfinder: Schmitz-Rode, Thomas, Dr. med., W-5100 Aachen (DE)
(74) Vertreter: Dipl.-Ing. Heiner Lichti Dipl.-Phys. Dr.rer.nat. Jost Lempert Dipl.-Ing. Hartmut Lasch

(57) **Zusammenfassung**

Die Erfindung betrifft eine Vorrichtung zum Entfernen von organischen Materialien aus dem menschlichen Körper mittels eines Katheters (1) mit einer Saugvorrichtung und einer im Katheter befindlichen Sonde (3). Zur Verhinderung eines Verstopfens des Katheters durch das abgesaugte Material unter Vermeidung eines Erwärmens und Reduzierung der Materialermüdung sieht die Erfindung vor, daß die Sonde an ihrem proximalen Ende mit der Abtriebsachse eines Getriebes (6) (Schwingantrieb) verbunden ist, wobei das Getriebe die gleichförmige Bewegung des Motors (7) in eine Schwingungsbewegung seiner Abtriebsachse umsetzt, welche auf die Sonde übertragen wird.

## Beschreibung

Die Erfindung betrifft eine Vorrichtung zum Entfernen, insbesondere zum Absaugen von organischen Materialien aus dem menschlichen Körper, mit einem Katheter, welcher in seinem proximalen Abschnitt einen Seitenanschluß aufweist, der zur Materialabsaugung mit einem Unterdruck beaufschlagt wird, und einer in diesem Katheter befindlichen Sonde, deren proximaler Abschnitt aus dem proximalen Katheter durch eine Dichtung herausgeführt ist.

Die Entfernung von Blutgerinnseln aus dem Gefäßsystem oder von Blutergüssen oder Abszessen in Körperhöhlen stellt in vielen Fällen eine notwendige therapeutische Maßnahme dar. In der Regel erfolgt diese in Form eines chirurgischen Eingriffes.

Alternativ hierzu kann in das Blutgefäß oder in die Körperhöhle ein Katheter eingeführt werden, über den das Gerinnsel oder der Abszeßinhalt abgesaugt wird. Der Nachteil dieses Verfahrens liegt in der Verstopfung des Absaugkatheters durch Gerinnselfragmente, nekrotisches Gewebe oder fibrinöses Material. Hierdurch wird der transkatheterale Sog unwirksam und der Prozeß der Materialentfernung kommt zum Erliegen.

Zur Abhilfes dieses Problemes wurde in der Publikation von Schmitz-Rode und Günther: Ultrasound-assisted Aspiration Thrombectomy: In Vitro Investigations (Radiology 1991; 178:677-679) eine im Katheterlumen befindliche Metallsonde vorgeschlagen, die an ihrem proximalen Ende mit einem Ultraschall-Transducer gekoppelt ist. Während der Aspiration von Material verhindert die durch Ultraschall in Oszillationen versetzte Sonde, daß Fragmente im Katheterinneren akkumulieren können. Damit wird einer Verstopfung des Katheterlumens vorgebeugt. Ebenso wird eine Verlegung der Katheterspitze verhindert, sofern die oszillierende Sonde bis in den Katheterspitzenbereich vorgeschoben ist.

Das geschilderte Verfahren ermöglicht die kontinuierliche Aspiration großer Mengen von Blutgerinnseln durch relativ kleinlumige Katheter, ohne daß eine Katheterverstopfung auftritt. Der Nachteil dieses Verfahrens besteht darin, daß aufgrund der Materialbelastung durch die Ultraschallwellen nur relativ dicke Sonden verwendet werden können, die entsprechend unflexibel sind und beispielsweise gekrümmten Gefäßverläufen nicht folgen können. Außerdem heizen sich die Sonden während des Betriebes auf, so daß die potentielle Gefahr besteht, daß thermisch weniger resistente Kathetermaterialien schmelzen können oder gesundes Gewebe durch eine aus dem Katheter herausragende erhitzte Sondenspitze geschädigt werden kann. Durch die kombinierte thermische und mechanische Belastung bricht die Sonde vorzugsweise im proximalen Bereich. Ein zusätzlicher Kühlkreislauf für das besonders gefährdete proximale Sondenstück kompliziert die Apparatur und erschwert die Handhabung. Die relativ hohen Kosten für die Ultraschallapparatur stellen einen weiteren Nachteil dar.

Der Erfindung liegt die Aufgabe zugrunde, ein Kathetersystem zur kontinuierlichen Absaugung von organischen Materialien aus dem menschlichen Körper der eingangs beschriebenen Art bereitzustellen, welches keine thermischen Nebeneffekte besitzt, welches auch bei längerer Betriebsdauer keine Materialermüdung aufweist, welches flexibel genug ist, um auch in gekrümmte Körpersegmente eingesetzt werden zu können und außerdem relativ kostengünstig herstellbar ist.

Zur Lösung der Aufgabe wird eine Vorrichtung der eingangs genannten Art dadurch gebildet, daß die Sonde an ihrem proximalen Ende mit der Abtriebsachse eines Getriebes (Schwingteil) verbunden ist, wobei das Getriebe die gleichförmige Bewegung des Motors in eine Schwingungsbewegung seiner Abtriebsachse umsetzt, welche auf die Sonde übertragen wird.

Durch Verbindung des proximalen Endes der aus dem proximalen Katheterende herausragenden Sonde mit der Abtriebsachse eines Getriebes, welches die Motorbewegung in eine Schwingungsbewegung umwandelt, wird die Sonde innerhalb des Katheters ebenso in Schwingungen versetzt, wobei die Schwingungsfrequenz jedoch deutlich unterhalb des Ultraschallbereiches liegen kann. Eine wesentliche Erwärmung der Sonde erfolgt mit dieser Anordnung auch bei längerer Betriebsdauer nicht, so daß sich unerwünschte thermische Effekte wie die Schädigung des Katheters oder gar Schädigung gesunden Gewebes nicht einstellen können. Ebenso ergibt sich auch bei längerem Betrieb keine wesentliche Belastung des Sondenmateriales, welche einen Sondenbruch zur Folge haben könnte. Des weiteren kann insbesondere für den intraversalen Einsatz der Durchmesser der Sonde unter Beibehaltung des Wirkungsprinzips verringert werden. Eine solche dünne Sonde ist flexibler und läßt sich auch in gekrümmten Gefäßverläufen einsetzen. Schließlich ist eine Motor-Getriebe-Einheit für diesen Anwendungsfall erheblich kostengünstiger herzustellen als die bisher verwendete Ultraschallapparatur.

Durch die Schwingbewegungen der länglichen Sonde wird die Akkumulation von Fragmenten im Körperinneren zuverlässig vermieden.

Die Sonde ist ein längliches Drahtteil, vorzugsweise aus Metall, wie rostfreiem Stahl; sie kann aber auch aus nichtmetallischem Material bestehen, wie Glas- oder Graphitfaser, oder aus Kunststoff, gegebenenfalls faserverstärkt.

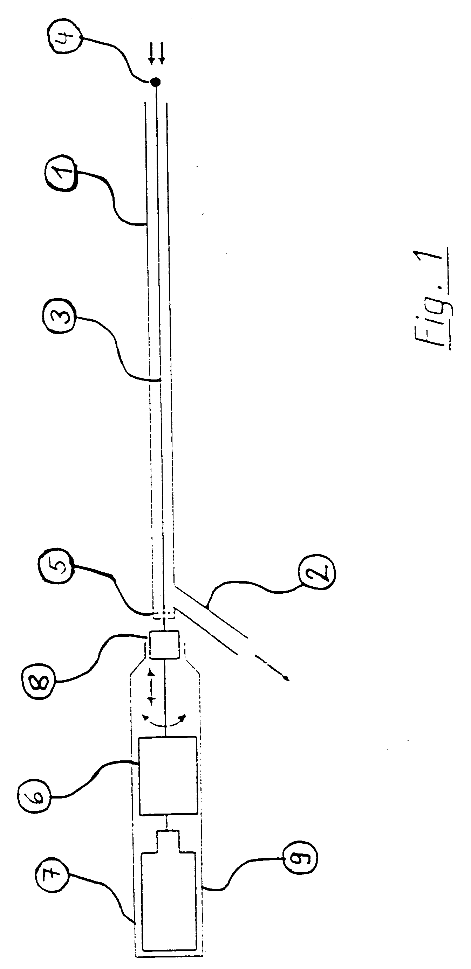

Die Erfindung wird anhand Figur 1 erläutert:
Ein dünnwandiger großlumiger Katheter 1 besitzt in seinem proximalen, also rückwärtigen Abschnitt einen Seitenanschluß 2, der mit einem Sog beaufschlagt wird (durch Anschluß an eine Saugpumpe oder durch Konnektieren einer Saugspritze). Innerhalb des Katheters 1 befindet sich eine Sonde 3, deren Durchmesser deutlich kleiner ist als das Katheter-Innenlumen und deren von der Sonde distales Ende 4 je nach Anforderung verschieden gestaltet sein kann (z.B. Kugelkopf, Öse, Spirale etc.). Das vordere Ende lockert Ablagerungen im Gefäß, so daß die Fragmente dann in den Katheter eingesaugt werden können. Der proximale Sondenanteil ist durch eine Dichtung 5 aus dem proximalen Katheterende herausgeführt und über einen Konnektor 8 mit einem durch einen Motor 7 angetriebenen Getriebe 6 als Schwingantrieb verbunden. Das Getriebe 6 (z.B. Koppel- oder Kurvengetriebe) setzt die Motorenergie um in longitudinale und eventuell zusätzlich rotatorische Schwingungen seiner Abtriebsachse, und dies mit einer Frequenz, die deutlich unter dem Ultraschallbereich liegt. Über den Konnektor 8 werden diese Schwingungen der Abtriebsachse des Getriebes 6 auf die im Katheterlumen befindliche Sonde 3 übertragen. Zur Erleichterung der Handhabung können Motor 7, Getriebe 6 und Konnektor 8 in einem Gehäuse 9 zusammengefaßt sein, welches während des Betriebes in der Hand gehalten werden kann.

## Patentansprüche

1. Vorrichtung zum Entfernen, insbesondere zum Absaugen von organischen Materialien aus dem menschlichen Körper, mit einem Katheter, welcher in seinem proximalen Abschnitt einen Seitenanschluß aufweist, der zur Materialabsaugung mit einem Unterdruck beaufschlagt wird, und einer in diesem Katheter befindlichen Sonde, deren proximaler Abschnitt aus dem proximalen Katheterende durch eine Dichtung herausgeführt ist,
dadurch gekennzeichnet,
daß die Sonde (3) an ihrem proximalen Ende mit der Abtriebsachse eines Getriebes (6) (Schwingantrieb) verbunden ist, wobei das Getriebe (6) die gleichförmige Bewegung des Motors (7) in eine Schwingungsbewegung seiner Abtriebsachse umsetzt, welche auf die Sonde (3) übertragen wird.

2. Vorrichtung nach Anspruch 1, dadurch gekennzeichnet, daß das Getriebe als longitudinaler Schwingantrieb der Sonde Longitudinalbewegungen aufweist.
